(19) Europäisches Patentamt / European Patent Office / Office européen des brevets

(11) **EP 3 005 930 A1**

(12) **EUROPEAN PATENT APPLICATION**
published in accordance with Art. 153(4) EPC

(43) Date of publication:
**13.04.2016 Bulletin 2016/15**

(51) Int Cl.:
*A61B 1/00* (2006.01)  *G02B 6/255* (2006.01)
*G02B 6/36* (2006.01)

(21) Application number: **14804662.6**

(22) Date of filing: **29.05.2014**

(86) International application number:
**PCT/JP2014/064255**

(87) International publication number:
**WO 2014/192866 (04.12.2014 Gazette 2014/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.05.2013 JP 2013113198**

(71) Applicants:
• **Sumitomo Electric Industries, Ltd.**
  **Osaka-shi, Osaka 541-0041 (JP)**
• **SEI Optifrontier Co., Ltd.**
  **Yokohama-shi, Kanagawa 244-8589 (JP)**

(72) Inventors:
• **MURASHIMA, Kiyotaka**
  **Yokohama-shi**
  **Kanagawa 244-8588 (JP)**
• **YAMAGUCHI, Ryo**
  **Yokohama-shi**
  **Kanagawa**
  **2448588 (JP)**
• **FUKUI, Junji**
  **Chigasaki-shi**
  **Kanagawa 253-0087 (JP)**

(74) Representative: **Hoffmann Eitle**
  **Patent- und Rechtsanwälte PartmbB**
  **Arabellastraße 30**
  **81925 München (DE)**

(54) **CATHETER FOR OPTICAL COHERENCE TOMOGRAPH, AND CATHETER PRODUCTION METHOD**

(57) An OCT catheter and a method for manufacturing the OCT catheter, with which the operation time, manufacturing cost, and the risk of breakage of a spliced portion between a short-length fiber and an optical fiber contained in an interior member can be reduced, are provided. An OCT catheter 1 A includes an interior member 10 that contains an optical fiber and that is to be inserted into a body, a metal tube 20 that guides the interior member 10, and an optical connector 30. The optical connector 30 includes a ferrule assembly including a short-length fiber and a ferrule fixed to one end of the short-length fiber, and is connectable to a rotary joint of an OCT system. The other end of the short-length fiber and one end of the optical fiber contained in the interior member 10 are fusion-spliced together to form a spliced portion. The spliced portion is disposed in the metal tube 20.

FIG. 1

EP 3 005 930 A1

## Description

Technical Field

The present invention relates to an optical coherence tomography catheter and a method for manufacturing the optical coherence tomography catheter.

Background Art

US Patent No. 6,445,939 (Patent Document 1) discloses a method for connecting a catheter for an optical coherence tomography (OCT) system, which images the inside of a cavity, to a rotary joint. An FC/APC connector is used in this method. US Patent No. 7,813,609 (Patent Document 2) describes the structure of a catheter for an OCT system. This catheter includes an interior member that rotates, a tube housing that covers the interior member, and an optical connector that provides connection to a rotary joint.

According to the OCT catheters of the related art described in Patent Documents 1 and 2, a method of assembling a connecting portion to be connected to the rotary joint includes attaching a ferrule to a proximal end of an optical fiber contained in the interior member, and polishing an end surface of the optical fiber at an end surface of the ferrule. The interior member is generally expensive, and therefore needs to be processed carefully. Therefore, the process takes a long time and the manufacturing cost is high.

Japanese Unexamined Patent Application Publication No. 2009-205100 (Patent Document 3), Japanese Unexamined Patent Application Publication No. 2011-118348 (Patent Document 4), and Japanese Unexamined Patent Application Publication No. 2008-197622 (Patent Document 5) describe so-called field-installable fusion splice single-core optical connectors. The field-installable fusion splice single-core optical connectors can be attached to an optical fiber in the field, and include a ferrule and a short-length fiber fitted to the ferrule. A ferrule-side end (a front end) of the short-length fiber is polished in advance, and a rear end of the short-length fiber is fusion-spliced to a front end of the optical fiber to be connected.

Summary of Invention

Technical Problem

An object of the present invention is to provide an OCT catheter and a method for manufacturing the OCT catheter with which the assembly time, manufacturing cost, and the risk of breakage of a spliced portion between a short-length fiber and an optical fiber contained in an interior member can be reduced.

Solution to Problem

To achieve the above-described object, a method for manufacturing an optical coherence tomography catheter is provided. The optical coherence tomography catheter includes an interior member that contains a first optical fiber and that is to be inserted into a body, and an optical connector including a ferrule assembly, which includes a second optical fiber and a ferrule fixed to one end of the second optical fiber, and a tube, one end of which is directly or indirectly fixed to the ferrule assembly and which guides the interior member, the optical connector being connectable to a rotary joint of an optical coherence tomography system. The method includes a first step of selecting the ferrule assembly including an embedded optical fiber having a first length as the second optical fiber, cutting the first optical fiber so that a sum of lengths of the first optical fiber and the second optical fiber is equal to a predetermined length, and fusion-splicing one end of the first optical fiber and the other end of the second optical fiber together to form a spliced portion; and a second step of inserting the spliced portion into the tube. When the cutting of the optical fiber or the fusion-splicing fails in the first step, the ferrule assembly including an embedded optical fiber having a second length, which is greater than the first length, as the second optical fiber is selected, the first optical fiber is cut again, and the spliced portion is formed again.

According to another aspect of the present invention, an optical coherence tomography catheter is provided. The optical coherence tomography catheter includes (1) an interior member that contains a first optical fiber and that is to be inserted into a body; (2) an optical connector including a ferrule assembly, which includes a second optical fiber and a ferrule fixed to one end of the second optical fiber, and a tube, one end of which is directly or indirectly fixed to the ferrule assembly and which guides the interior member, the optical connector being connectable to a rotary joint of an optical coherence tomography system; and (3) a spliced portion in which the other end of the second optical fiber and one end of the first optical fiber are fusion-spliced together, the spliced portion being disposed in the tube.

Advantageous Effects of Invention

According to the OCT catheter and the method for manufacturing the OCT catheter of the present invention, the assembly time, manufacturing cost, and the risk of breakage of the spliced portion between the short-length fiber and the optical fiber contained in the interior member can be reduced.

Brief Description of Drawings

Figure 1 is a perspective view of an OCT catheter according to an embodiment, illustrating the state in which a tube housing is removed.

Figure 2 is an exploded perspective view of an optical connector.

Figure 3 is a sectional view of the optical connector in a longitudinal direction.

Figure 4 is an enlarged sectional view illustrating the detailed structures of a ferrule assembly and a joint member.

Figure 5 is a diagram illustrating the shape of the joint member, wherein Fig. 5(a) is a front view, Fig. 5(b) is a side view, Fig. 5(c) is a rear view, and Fig. 5(d) is a sectional side view.

Figure 6 is a sectional view of the optical connector in the longitudinal direction according to a modification.

Figure 7 is a sectional view of the optical connector in the longitudinal direction according to another modification.

Figure 8 is a conceptual diagram illustrating a spliced portion formed by fusion-splicing a proximal end of an optical fiber contained in an interior member and the other end of a short-length fiber.

Figure 9 is a flowchart of a method for manufacturing the OCT catheter.

Figure 10 is a conceptual diagram illustrating the interior member, a metal tube, and the ferrule assembly.

Figure 11 is a conceptual diagram illustrating another example of a structure for fixing the ferrule assembly to the metal tube as a modification.

Figure 12 is a conceptual diagram illustrating the structure of a joint member.

Figure 13 is a sectional view illustrating the structure of a joint member.

Description of Embodiments

The inventor of the present invention has considered applying a field-installable fusion splice single-core optical connector to a catheter for an OCT system. This eliminates the necessity of a polishing step in the assembly of the catheter. In addition, the field-installable fusion splice single-core optical connector is inexpensive, and therefore the manufacturing cost can be reduced. However, the method using the field-installable fusion splice single-core optical connector involves the following problems.

That is, OCT catheters are required to include an optical fiber having a predetermined optical path length so that interference waves can be observed. The allowable error of the optical path length is, for example, as small as ±5 mm or ±2 mm. Field-installable fusion splice single-core optical connectors include a short-length fiber having a constant length. If a spliced portion between the short-length fiber and an optical fiber contained in an interior member breaks, the length of the optical fiber contained in the interior member is slightly reduced when the optical fibers are fusion-spliced together again. Therefore, there is a risk that the error in the optical path length will be greater than the allowable error.

An OCT catheter and a method for manufacturing the OCT catheter according to an embodiment of the present invention will be described with reference to the accompanying drawings. In the drawings, the same components are denoted by the identical reference numerals, and redundant descriptions are omitted.

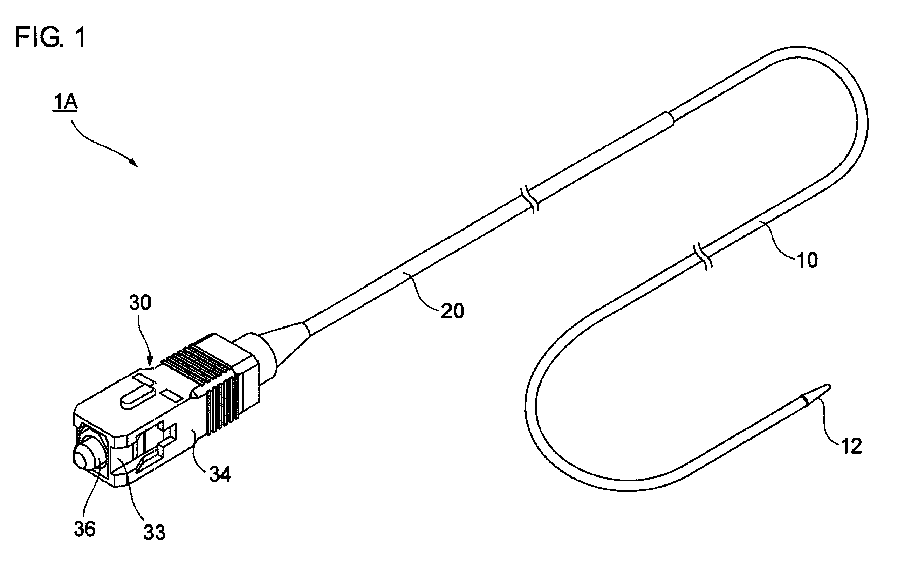

Figure 1 is a perspective view of an OCT catheter 1A according to an embodiment of the present invention, illustrating the state in which a tube housing is removed. The OCT catheter 1A includes an interior member (torque wire) 10, which is to be inserted into a body, and an optical connector 30 to be connected to a rotary joint of an OCT system. A lens 12, through which light is emitted toward the inside of the body and interference light is received, is attached to the distal end of the interior member 10. The interior member 10 contains an optical fiber (first optical fiber) that optically couples the optical connector 30 to the lens 12. A proximal end portion of the interior member 10 (portion that is not inserted into the body) is inserted into a metal tube 20, which will be described below, at one end of the metal tube 20 and is held in such a manner that the proximal end portion is movable in a longitudinal direction.

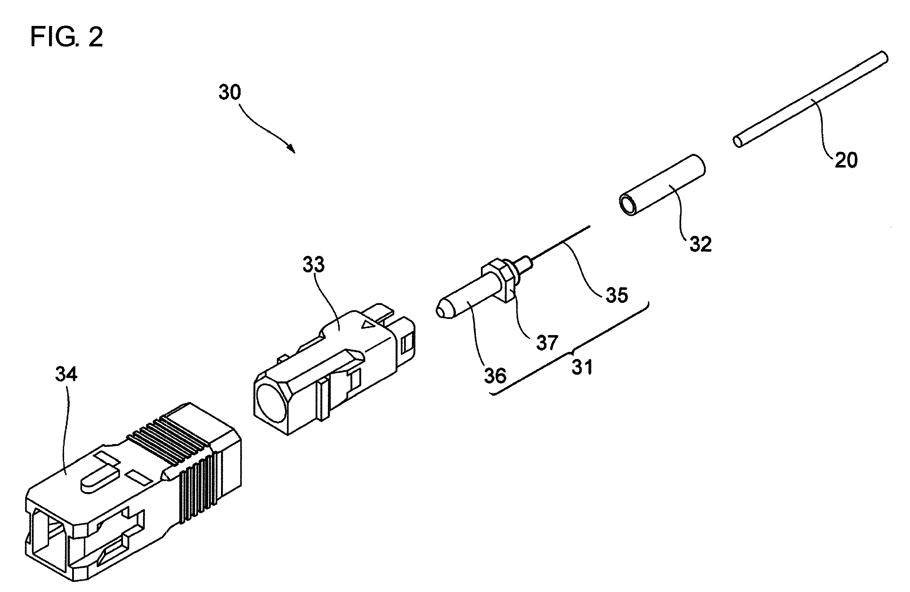

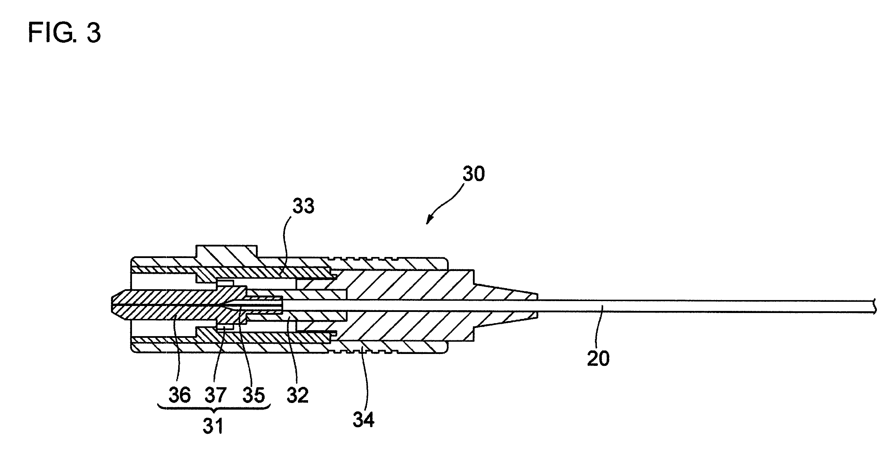

Figure 2 is an exploded perspective view of the optical connector 30. Figure 3 is a sectional view of the optical connector 30 in the longitudinal direction. The optical connector 30 is a so-called fusion splice SC connector. The optical connector 30 includes a ferrule assembly 31; the metal tube 20, one end of which is directly or indirectly fixed to the ferrule assembly 31 and which guides the interior member 10; a joint member 32 with which the metal tube 20 is fixed to the ferrule assembly 31; a plug housing 33 that accommodates the ferrule assembly 31 and the joint member 32, and an outer housing 34 that covers the plug housing 33. The metal tube 20 guides the interior member 10 in the longitudinal direction during, for example, a pull-back operation, and holds the proximal end portion of the interior member 10 so that the proximal end portion is not bent.

The ferrule assembly 31 includes a short-length fiber (also referred to as a second optical fiber or an embedded optical fiber) 35; a substantially columnar ferrule 36 that is fixed to one end of the short-length fiber 35 and holds the short-length fiber 35; and a tubular metal flange member 37 that is fixed to the ferrule 36. The ferrule 36 holds the short-length fiber 35 such that the short-length fiber 35 extends along the center axis of the ferrule 36. An end surface (front end surface) of the ferrule 36 is polished so as to be at an angle (for example, 8 degrees) with respect to a plane perpendicular to the center axis of the short-length fiber 35.

The short-length fiber 35 projects from the ferrule assembly 31 into the metal tube 20 by a predetermined length. The proximal end surface of the optical fiber contained in the interior member 10 is fusion-spliced to the metal-tube-20-side end surface of the short-length fiber 35. As described below, the spliced portion between the optical fiber contained in the interior member 10 and the short-length fiber 35 is disposed in the metal tube 20.

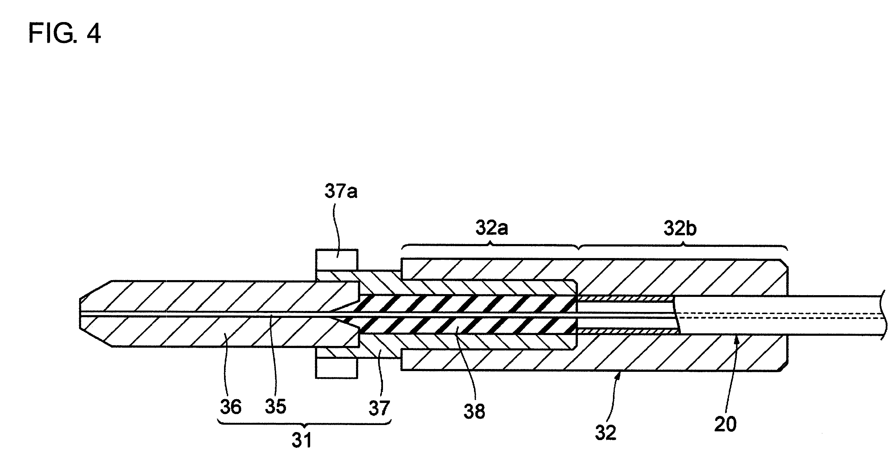

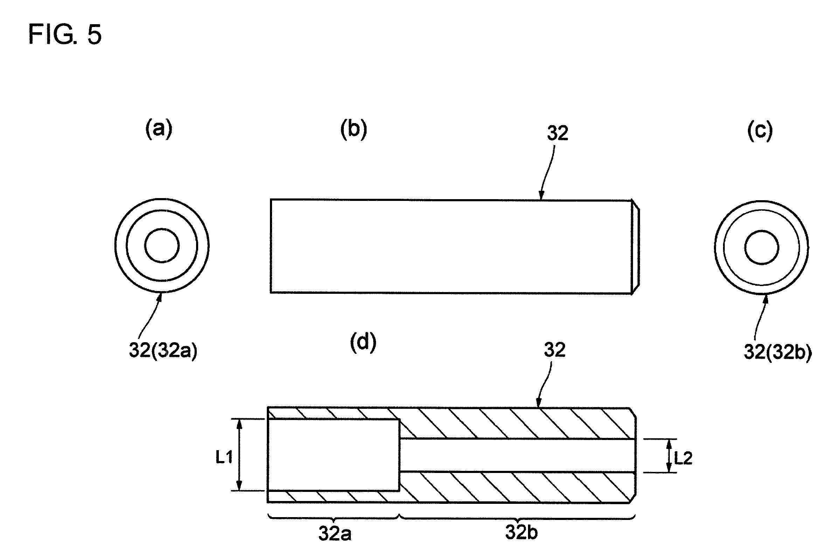

Figure 4 is an enlarged sectional view illustrating the detailed structures of the ferrule assembly 31 and the joint member 32. Figure 5 is a diagram illustrating the shape of the joint member 32, wherein Fig. 5(a) is a front view, Fig. 5(b) is a side view, Fig. 5(c) is a rear view, and Fig. 5(d) is a sectional side view.

The flange member 37 has a cylindrical shape and extends in the axial direction of the short-length fiber 35, and one end of the flange member 37 is securely fitted to the other end portion of the ferrule 36. The short-length fiber 35 extends through the flange member 37. The flange member 37 includes a flange 37a that projects in a direction that crosses the axial direction of the short-length fiber 35. The flange 37a is brought into contact with a projection formed on the inner wall surface of the plug housing 33, so that the ferrule assembly 31 is positioned. The inner diameter of the flange member 37 is sufficiently greater than the diameter of the short-length fiber 35. The gap between the flange member 37 and the short-length fiber 35 is filled with a resin 38 that holds the short-length fiber 35. The resin 38 is composed of a resin material such as an epoxy resin or an acrylic resin.

The joint member 32 is provided to fix the metal tube 20 to the ferrule assembly 31. As illustrated in Fig. 5, the joint member 32 has a substantially cylindrical shape, and includes a front portion 32a having a first inner diameter L1 on one side thereof and a rear portion 32b having a second inner diameter L2, which is smaller than the first inner diameter L1, on the other side thereof. The other end portion of the flange member 37 is inserted into the front portion 32a, so that the joint member 32 and the flange member 37 are securely fitted together. The proximal end portion of the metal tube 20 is inserted into the rear portion 32b. (The rear portion 32b of the joint member 32, that is, a portion of the joint member 32 into which the metal tube 20 is inserted, has a constant inner diameter and a constant outer diameter. In other words, the area of the cross section of this portion that is perpendicular to the longitudinal direction is constant.) The short-length fiber 35 extends through the front portion 32a and the rear portion 32b. The joint member 32 is made of a material such as SUS304.

The length of the joint member 32 in the axial direction of the short-length fiber 35 is preferably such that the joint member 32 can be disposed in the plug housing 33. However, as long as the joint member 32 is disposed in a bonding member (member that covers the optical connector 30 that rotates at a high speed) that is bonded to the rotary joint, the joint member 32 may have a length such that the joint member 32 projects from the plug housing 33.

When the OCT catheter 1A is used, the optical connector 30 is rotated at a high speed. The rotating force is applied to the interior member 10 through the metal tube 20. Depending on the material and structure of the metal tube 20, resonance may occur at the proper angular frequency. Therefore, the joint member 32 preferably has such a length that the resonance can be prevented.

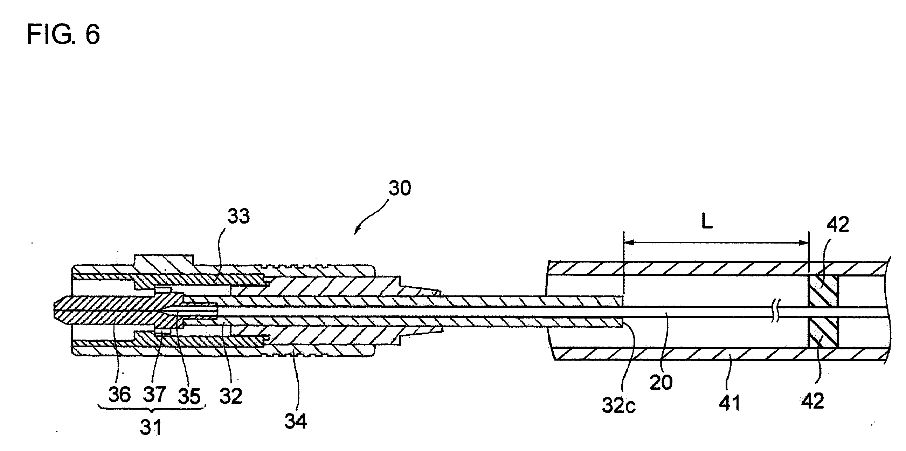

Figure 6 is a sectional view of the optical connector 30 in the longitudinal direction according to a modification. The OCT catheter 1A further includes a tube housing 41. The tube housing 41 is a tubular body that covers the interior member 10 and the optical connector 30. One end of the tube housing 41 is fixed to the OCT system. Although the interior member 10 and the optical connector 30 are rotated with respect to the OCT system, the tube housing is stationary and does not rotate. The gap between the tube housing 41 and the interior member 10 is filled with a liquid, such as a physiological saline solution.

A support 42, which supports the interior member 10 or the metal tube 20, is disposed in the tube housing 41. In many cases, the support 42 holds the metal tube 20. In one embodiment, the support 42 is a stop valve that prevents the above-described liquid from flowing into the optical connector 30. To prevent the metal tube 20 from resonating, the length of the joint member 32 is preferably set such that the distance L between the other end 32c of the joint member 32 and the support 42 is outside a range in which the metal tube 20 resonates when the rotational speed of the optical connector 30 is within an operational rotational speed range (for example, 0 rpm to 12000 rpm).

The distance L at which the metal tube 20 resonates can be determined as follows. That is, let L be the distance between the other end 32c of the joint member 32 and the support 42, E be the flexural rigidity (Young's modulus) of the metal tube 20, I be the second moment of area of the metal tube 20, i be the order number of the resonance, $\rho$ be the density of the metal tube 20, and A be the area of cross section of the metal tube 20 that is perpendicular to the center axis, the proper angular frequency $p_i$ of the metal tube 20 can be determined by the following Equation (1).

$$p_i = \left(\frac{i\pi}{L}\right)^2 \sqrt{\frac{EI}{\rho A}} \quad \cdots (1)$$

As the distance L increases, the resonant frequency decreases. In Equation (1), the range of the distance L at which the metal tube 20 resonates can be determined by substituting the operational rotation frequency of the optical connector 30 for the proper angular frequency $p_i$ and calculating the distance L corresponding to the operational rotation frequency. The length of the joint member 32 is preferably set such that the distance L is outside the range. Since the support 42 is fixed to the tube housing 41, the support 42 is stationary during the pull-back operation of the interior member 10. Therefore, the distance L changes as the joint member 32 is moved in the axial direction in the pull-back operation. Such a change is preferably taken into consideration when determining the range of the distance L. More specifically, the distance of the joint member 32 is preferably determined so that the proper angular frequency $p_i$ is greater than or equal to 1.25 times the operational rotation frequency, in other words, so that the operational rotation frequency is smaller than 0.8 times the proper angular frequency. More preferably, the metal tube 20 is made of a material that is not easily plastically deformed (for example, SUS304) to reduce damage to the metal tube 20 caused by the resonance.

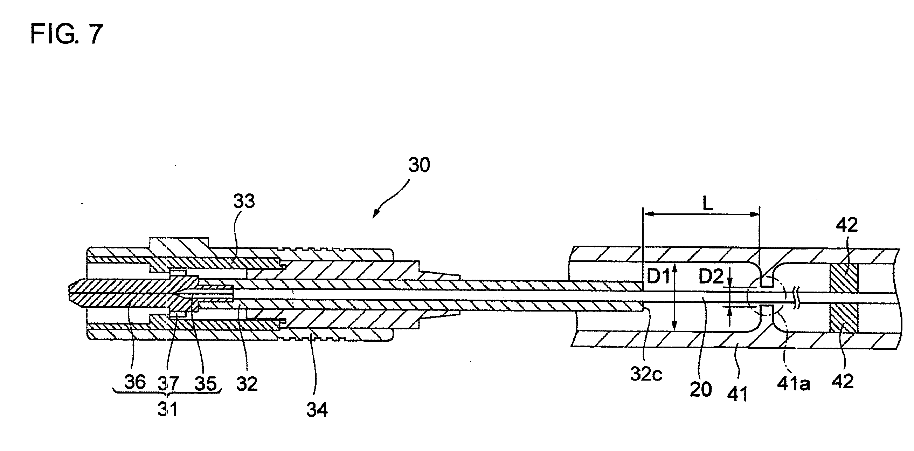

Figure 7 is a sectional view of the optical connector 30 in the longitudinal direction according to another modification. The tube housing 41 includes a portion having a first inner diameter D1 and a portion (narrow portion) having a second inner diameter D2 that is smaller than the first inner diameter D1 in a region between the other end 32c of the joint member 32 and the support 42. In this case, the narrow portion may be regarded as another support 41 a. The distance L may be determined as the distance between the other end 32c of the joint member 32 and the support 41 a.

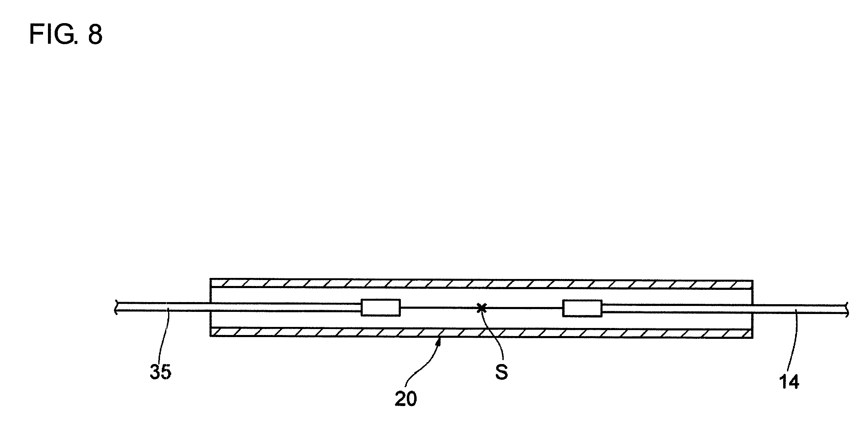

Figure 8 is a conceptual diagram illustrating a spliced portion S formed by fusion-splicing the proximal end of the optical fiber 14 (first optical fiber) contained in the interior member 10 and the other end of the short-length fiber 35 (second optical fiber). The OCT catheter 1A further includes the spliced portion S. The spliced portion S is disposed in the metal tube 20, and is protected by the metal tube 20 so that breakage thereof does not occur. Preferably, a protecting member for protecting a glass fiber included in the optical fiber 14 and a glass fiber included in the short-length fiber 35 is provided in a section where the glass fibers are exposed. The protecting member may have various structures, such as a tube fixed with an adhesive, a heat-shrinkable tube, or a curing agent applied by using a pen or the like.

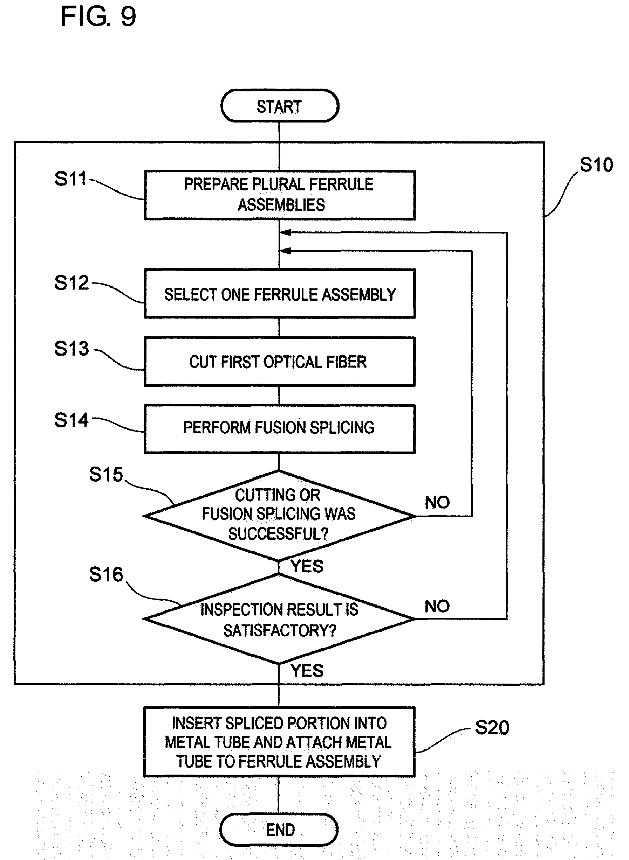

Figure 9 is a flowchart of a method for manufacturing the OCT catheter 1A. In a first step S10, a plurality of ferrule assemblies 31 including short-length fibers 35 having different lengths are prepared (step S11). Next, among the ferrule assemblies 31, a ferrule assembly 31 including a short-length fiber 35 having a certain length (first length) is selected (step S12). Then, the optical fiber 14 is cut so that the sum of the lengths of the optical fiber 14 and the short-length fiber 35 is equal to a predetermined length, that is, an optical path length suitable for observation of interference waves (step S 13). Then, one end of the optical fiber 14 and the other end of the short-length fiber 35 are fusion-spliced together to form the spliced portion S (step S 14).

In the first step S10, there is a possibility that the process of cutting the optical fiber 14 (step S 13) or the fusion splicing process (step S14) will fail (No in step S15). In such a case, a ferrule assembly 31 including a short-length fiber 35 having a second length, which is longer than the first length, is selected (step S12). Then, the optical fiber 14 is cut again so that the sum of the lengths of the optical fiber 14 and the short-length fiber 35 is equal to the predetermined length (step S 13), and the spliced portion S is formed again (step S 14).

In the first step S10, the optical path length of the optical fiber 14 and the short-length fiber 35 or the fusion-spliced state of the spliced portion S may be inspected (step S16). Also when the inspection result shows that the optical path length of the optical fiber 14 and the short-length fiber 35 is outside the allowable error range or that the fusion-spliced state is not satisfactory, a ferrule assembly 31 including a short-length fiber 35 having the second length, which is longer than the first length, is selected (step S12). Then, the optical fiber 14 is cut again so that the sum of the lengths of the optical fiber 14 and the short-length fiber 35 is equal to the predetermined length (step S13), and the spliced portion S is formed again (step S 14).

After the first step S10, in a second step S20, the spliced portion S is inserted into the metal tube 20, and the metal tube 20 is attached to the ferrule assembly 31 by using the joint member 32. After that, the ferrule assembly 31 is placed in the plug housing 33, and the outer housing 34 is attached. Thus, the OCT catheter 1A is completed.

The advantageous effects of the above-described OCT catheter 1A and the method for manufacturing the OCT catheter 1A will be described. According to the OCT catheter 1A and the assembly method thereof, an optical fiber having a ferrule-side end surface that is polished in advance may be used as the short-length fiber 35, and it is not necessary to directly polish the optical fiber 14 contained in the interior member 10. Therefore, the assembly process can be easily carried out in a short time. In addition, a component of an SC optical connector may be used as the ferrule assembly 31 including the short-length fiber 35, so that the manufacturing cost may be reduced.

In addition, in the OCT catheter 1A, the spliced portion S is disposed in the metal tube 20. In the method for manufacturing the OCT catheter 1A, the spliced portion S is inserted into the metal tube 20 in the second step S20. Thus, the spliced portion S is effectively protected by the metal tube 20, and the risk of breakage of the spliced portion S can be reduced.

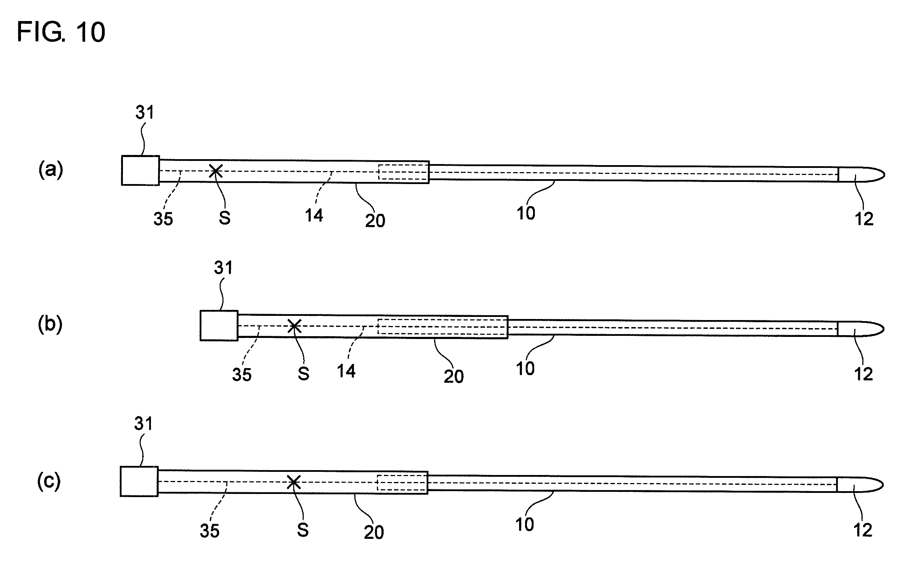

When a component of an SC optical connector is simply used as the ferrule assembly 31, the following problem occurs. Figure 10 is a conceptual diagram illustrating the problem, and schematically illustrates the interior member 10, the metal tube 20, and the ferrule assembly 31. As illustrated in Fig. 10(a), assume that the short-length fiber 35 and the optical fiber 14 are fusion-spliced together to form the spliced portion S. If the fusion splicing process fails, the optical fiber 14 needs to be cut and fusion-spliced again to the short-length fiber 35 of another ferrule assembly 31. However, if the optical fiber 14 becomes too short as a result of the process of cutting the optical fiber 14, as illustrated in Fig. 10(b), the sum of the optical path lengths of the optical fiber 14 and the short-length fiber 35 becomes smaller than the predetermined length. Such an interior member 10 cannot be used. The interior member 10 is very expensive, and it is preferable to avoid the situation where the interior member 10 becomes unusable. The above-described problem also occurs when the process of cutting the optical fiber 14 fails.

Accordingly, in the manufacturing method of the present embodiment, when the process of cutting the optical fiber 14 or the fusion splicing process fails in the first step S10, a ferrule assembly 31 including a longer short-length fiber 35 is selected. Then, the optical fiber 14 is cut and the spliced portion S is formed again. Accordingly, as illustrated in Fig. 10(c), the sum of the optical path lengths of the optical fiber 14 and the short-length fiber 35 may be set to the predetermined length, and the interior member 10 can be used continuously.

In addition, in the OCT catheter 1A, the optical connector 30 includes the tubular flange member 37, one end of which is fixed to the ferrule 36 such that the short-length fiber 35 extends therethrough, and the tubular joint member 32, one end of which is fitted to the other end of the flange member 37. The metal tube 20 is inserted into and fixed to the joint member 32 at the other end of the joint member 32. Since the OCT catheter 1A is structured as described above, the metal tube 20 can be securely fixed and does not easily come into contact with the spliced portion S, so that the risk of breakage of the spliced portion S can be further reduced.

The OCT catheter 1A includes the tube housing that covers the interior member and the optical connector and that is stationary when the interior member and the optical connector rotate, and a support that is attached to an inner section of the tube housing and that supports the tube. The length of the joint member 32 is set such that the distance L between the other end 32c of the joint member 32 and the support 42 is outside the range in which the metal tube 20 resonates when the rotational speed of the optical connector 30 is within the operational rotational speed range. Thus, vibration due to resonance of the metal tube 20 can be prevented and burden on the subject can be reduced.

First Modification

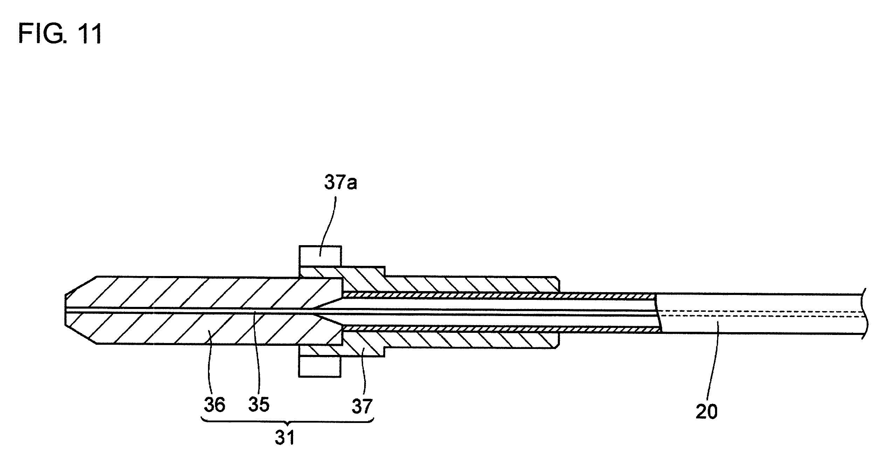

Figure 11 is a conceptual diagram illustrating another example of a structure for fixing the ferrule assembly 31 to the metal tube 20 as a modification of the above-described embodiment. In this modification, the gap between the flange member 37 and the short-length fiber 35 is not filled with a resin, and the metal tube 20 is inserted into and fixed to the flange member 37. One end of the metal tube 20 is in contact with the other end surface of the ferrule 36. Also in this case, similar to the above-described embodiment, the assembly process can be easily carried out in a short time. In addition, the manufacturing cost can be reduced. Furthermore, since the spliced portion S is disposed in the metal tube 20, the spliced portion S is effectively protected by the metal tube 20, and the risk of breakage of the spliced portion S can be reduced.

The flange member 37 included in the ferrule assembly 31 for an SC connector is short, and therefore there may be a case in which the metal tube 20 cannot be securely fixed to the flange member 37. When the metal tube 20 cannot be securely fixed, there is a risk that the metal tube 20 will become unstable during high-speed rotation of the OCT catheter 1 A. In such a case, the ferrule assembly 31 and the metal tube 20 may be fixed to each other by using the joint member 32 as in the above-described embodiment. Second Modification

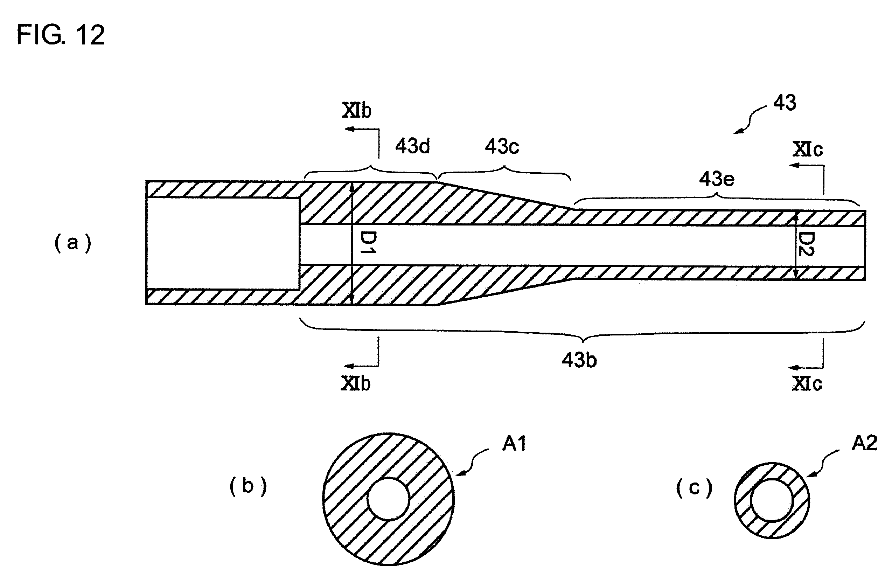

Figure 12 is a conceptual diagram illustrating the shape of a joint member 43 as another modification of the above-described embodiment. Figure 12(a) is a sectional side view of the joint member 43, Fig. 12(b) is a sectional view taken along line XIb-XIb, and Fig. 12(c) is a sectional view taken along line XIc-XIc. The optical connector 30 may include the joint member 43 instead of the joint member 32. The joint member 43 includes a rear portion 43b into which the metal tube 20 is inserted. The area of a cross section A2 of the rear portion 43b at the other-end side (Fig. 12(c)), the cross section A2 being perpendicular to the axial direction of the metal tube 20, is smaller than the area of a cross section A1 of the rear portion 43b at the one-end side (Fig. 12(b)), the cross section A1 being perpendicular to the axial direction of the metal tube 20. More specifically, a tapered portion 43c having an outer diameter that gradually decreases from one end toward the other end is disposed between the one end and the other end of the rear portion 43b. A portion 43d between the tapered portion 43c and the one end has an outer diameter D1, and a portion 43e between the tapered portion 43c and the other end has an outer diameter D2 (< D1).

In the metal tube 20, stress concentration occurs and maximum bending stress is applied at the boundary between the portion held by the joint member and the portion that is not held (that is, in a region near the other end of the joint member). In this modification, the flexural rigidity of the rear portion of the joint member decreases toward the other end, so that the stress concentration on the metal tube 20 can be reduced and damage to the metal tube 20 can be reduced accordingly.

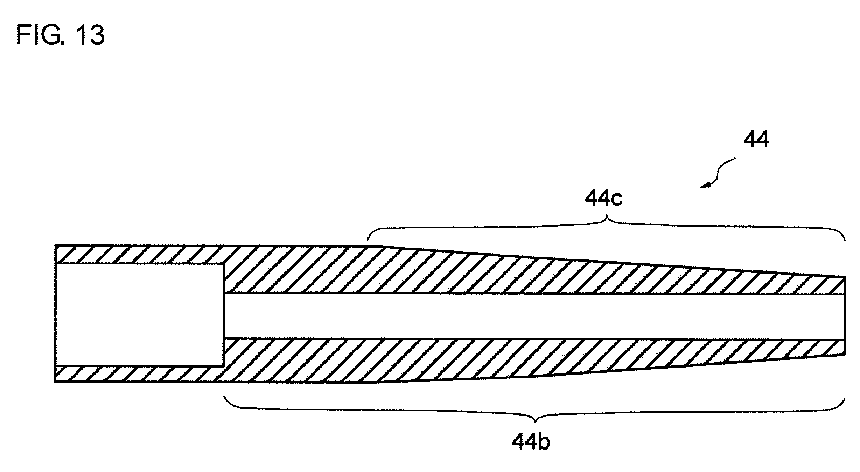

The shape of the joint member according to the present modification is not limited to that illustrated in Fig. 12. Figure 13 is a conceptual diagram illustrating a joint member 44 having another shape according to the present modification, and is a sectional side view of the joint member 44 in the axial direction. The joint member 44 includes a rear portion 44b including a tapered portion 44c that extends to the other end of the joint member 44. Also in this case, the effects of the present modification can be obtained.

The OCT catheter and the method for manufacturing the OCT catheter according to preferred embodiments of the present invention have been described. However, the present invention is not limited to the above-described embodiments, and various changes may be made without departing from the scope thereof. For example, in the above-described embodiments, the optical connector is a fusion splice SC connector. However, the optical connector may instead be a fusion splice connector other than the fusion splice SC connector. In addition, although a metal tube is described as an example of a tube that guides the interior member in the above-described embodiments, the tube may instead be made of other materials, such as a resin.

## Claims

1. A method for manufacturing an optical coherence tomography catheter including
   an interior member that contains a first optical fiber and that is to be inserted into a body, and
   an optical connector including a ferrule assembly, which includes a second optical fiber and a ferrule fixed to one end of the second optical fiber, and a tube, one end of which is directly or indirectly fixed to the ferrule assembly and which guides the interior member, the optical connector being connectable to a rotary joint of an optical coherence tomography system,
   the method comprising:
   a first step of selecting the ferrule assembly including an embedded optical fiber having a first length as the second optical fiber, cutting the first optical fiber so that a sum of lengths of the first optical fiber and the second optical fiber is equal to a predetermined length, and fusion-splicing one end of the first optical fiber and the other end of the second optical fiber together to form a spliced portion; and
   a second step of inserting the spliced portion into the tube,
   wherein, when the cutting of the optical fiber or the fusion-splicing fails in the first step, the ferrule assembly including an embedded optical fiber having a second length, which is greater than the first length, as the second optical fiber is selected, the first optical fiber is cut again, and the spliced portion is formed again.

2. An optical coherence tomography catheter comprising:

   an interior member that contains a first optical fiber and that is to be inserted into a body;
   an optical connector including a ferrule assembly, which includes a second optical fiber and a ferrule fixed to one end of the second optical fiber, and a tube, one end of which is directly or indirectly fixed to the ferrule assembly and which guides the interior member, the optical connector being connectable to a rotary joint of an optical coherence tomography system; and
   a spliced portion in which the other end of the second optical fiber and one end of the first optical fiber are fusion-spliced together, the spliced portion being disposed in the tube.

3. The optical coherence tomography catheter according to Claim 2,
   wherein the optical connector includes

   a tubular flange member, one end of which is fixed to the ferrule such that the second optical fiber extends through the tubular flange member, and
   a tubular joint member, h is fitted to the other end of the flange member,

   wherein the tube is inserted into and fixed to the joint member at the other end of the joint member.

4. The optical coherence tomography catheter according to Claim 3, further comprising:

   a tube housing that covers the interior member and the optical connector and that is stationary when the interior member and the optical connector rotate,

   wherein the tube housing has a support disposed therein, the support supporting the interior member or the tube, and a distance between the other end of the joint member and the support is outside a range in which the tube resonates when a rotational speed of the optical connector is within an operational rotational speed range.

5. The optical coherence tomography catheter according to Claim 3 or 4,
   wherein an area of a cross section of a portion of the joint member in which the tube is inserted, the cross section being perpendicular to an axial direction of the tube, is smaller at the other-end side of the portion of the joint member than at the one-end side of

**EP 3 005 930 A1**

the portion of the joint member.

FIG. 1

1A

10

12

20

30

34

33

36

FIG. 2

EP 3 005 930 A1

FIG. 3

FIG. 4

FIG. 5

(a)

(b)                                    32

(c)

32(32a)

32(32b)

(d)                                    32

L1

L2

32a          32b

FIG. 6

FIG. 7

EP 3 005 930 A1

FIG. 8

EP 3 005 930 A1

# FIG. 9

START

S10

S11 — PREPARE PLURAL FERRULE ASSEMBLIES

S12 — SELECT ONE FERRULE ASSEMBLY

S13 — CUT FIRST OPTICAL FIBER

S14 — PERFORM FUSION SPLICING

S15 — CUTTING OR FUSION SPLICING WAS SUCCESSFUL? — NO

YES

S16 — INSPECTION RESULT IS SATISFACTORY? — NO

YES

INSERT SPLICED PORTION INTO METAL TUBE AND ATTACH METAL TUBE TO FERRULE ASSEMBLY — S20

END

FIG. 10

(a)

31

35    S    14    20    10    12

(b)

31

35    S    14    20    10    12

(c)

31

35    S    20    10    12

EP 3 005 930 A1

FIG. 11

FIG. 12

(a)

(b)

(c)

EP 3 005 930 A1

FIG. 13

44

44c

44b

EP 3 005 930 A1

## INTERNATIONAL SEARCH REPORT

| | International application No. |
|---|---|
| | PCT/JP2014/064255 |

A.  CLASSIFICATION OF SUBJECT MATTER
*A61B1/00*(2006.01)i, *G02B6/255*(2006.01)i, *G02B6/36*(2006.01)i

According to International Patent Classification (IPC) or to both national classification and IPC

B.  FIELDS SEARCHED

Minimum documentation searched (classification system followed by classification symbols)
A61B1/00, G02B6/255, G02B6/36

Documentation searched other than minimum documentation to the extent that such documents are included in the fields searched
Jitsuyo Shinan Koho          1922-1996   Jitsuyo Shinan Toroku Koho   1996-2014
Kokai Jitsuyo Shinan Koho    1971-2014   Toroku Jitsuyo Shinan Koho   1994-2014

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y<br>A | JP 2009-240709 A  (Terumo Corp.),<br>22 October 2009 (22.10.2009),<br>paragraphs [0001] to [0011]; fig. 1 to 6<br>(Family: none) | 2<br>1,3-5 |
| Y<br>A | JP 2012-141437 A  (SEI Optifrontier Co., Ltd.),<br>26 July 2012 (26.07.2012),<br>paragraph [0019]; fig. 3<br>& US 2013/0230284 A1    & WO 2012/090570 A1 | 2<br>1,3-5 |
| Y<br>A | JP 2012-155316 A  (SEI Optifrontier Co., Ltd.),<br>16 August 2012 (16.08.2012),<br>paragraph [0019]; fig. 3<br>& US 2013/0276290 A1    & WO 2012/093566 A1 | 2<br>1,3-5 |

| ☒  Further documents are listed in the continuation of Box C. | ☐  See patent family annex. |
|---|---|

\*    Special categories of cited documents:
"A"   document defining the general state of the art which is not considered   to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search<br>    25 August, 2014 (25.08.14) | Date of mailing of the international search report<br>    02 September, 2014 (02.09.14) |
|---|---|
| Name and mailing address of the ISA/<br>    Japanese Patent Office<br><br>Facsimile No. | Authorized officer<br><br><br>Telephone No. |

Form PCT/ISA/210 (second sheet) (July 2009)

**INTERNATIONAL SEARCH REPORT**

International application No.

PCT/JP2014/064255

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2011-156217 A  (Hoya Corp.),<br>18 August 2011 (18.08.2011),<br>entire text; all drawings<br>(Family: none) | 1-5 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6445939 B
- US 7813609 B
- JP 2009205100 A
- JP 2011118348 A
- JP 2008197622 A